# EUROPÄISCHE PATENTSCHRIFT

(11) **EP 0 941 369 B1**
(45) Veröffentlichungstag und Bekanntmachung des Hinweises auf die Patenterteilung: **01.09.2004**
(21) Anmeldenummer: 97949919.1
(22) Anmeldetag: 12.11.1997
(51) Int. Cl.: C12Q 1/68

(54) **DNA ZUR ABSCHÄTZUNG DES PROGRESSIONSPOTENTIALS VON ZERVIXLÄSIONEN**
DNA FOR EVALUATING THE PROGRESSION POTENTIAL OF CERVICAL LESIONS
ADN PERMETTANT D'EVALUER LE POTENTIEL DE PROGRESSION DE LESIONS DU COL DE L'UTERUS

(30) Priorität: 27.11.1996 DE 19649207
(43) Veröffentlichungstag der Anmeldung: 15.09.1999
(73) Patentinhaber: Deutsches Krebsforschungszentrum Stiftung des öffentlichen Rechts, 69120 Heidelberg (DE)
(72) Erfinder: DÜRST, Matthias, D-69120 Heidelberg (DE); NEES, Matthias, D-69214 Eppelheim (DE)
(74) Vertreter: Schüssler, Andrea, Dr.
(86) Internationale Anmeldenummer: PCT/DE1997/002660
(87) Internationale Veröffentlichungsnummer: WO 1998/023775

(56) Entgegenhaltungen:
- WO-A-96/26293
- CHEN T.-M. ET AL.,: "Genetic analysis of in vitro progression of human papillomavirus transfected human cervical cells" CANCER RESEARCH, Bd. 53, - 1.März 1993 Seiten 1167-1171, XP002066423
- ODA D. ET AL.,: "Chromosomal abnormalities in HPV-16 immortalized oral epithelial cells" CARCINOGENESIS, Bd. 17, Nr. 9, - September 1996 Seiten 2003-2008, XP002066424
- DUERST M. ET AL.,: "malignat progression of an HPV-16 immortalizde human keratinocyte cell line (HPKIA) in vitro" CANCER GENET. CYTOGENET., Bd. 85, Nr. 2, - Dezember 1995 Seiten 105-112, XP002066425
- SEAGON S. & DUERST M.: "Genetic analysis of an in vitro model system for humanpapillomavirus type 16 associated tumorgenesis" CANCER RESEARCH, Bd. 54, - 1.November 1994 Seiten 5593-5598, XP002066426
- HESELMEYER ET AL.,: "Gain of chromosome 3q defines the transition from servere dysplasia to invasive carcinoma of the uterine cervix" PROC. NATL. ACAD. SCI. USA, Bd. 93, - Januar 1996 Seiten 479-484, XP002066427
- SOLINAS-TOLODO ET AL.,: "specific chromosomal imbalance in human papillomavirus transfected cells during progression toward immortality" PROC. NATL. ACAD. SCI. USA, Bd. 94, - April 1997 Seiten 3854-3859, XP002066428

## Beschreibung

Die Erfindung betrifft DNA, die sich zur Abschätzung des Progressionspotentials von Zervixläsionen eignet, und durch solche DNA codierte Polypeptide. Ferner betrifft die Erfindung Antikörper, die gegen die Polypeptide gerichtet sind. Des weiteren betrifft sie die Verwendung der DNA und der Polypeptide sowie einen zur Abschätzung der Progression von Zervixläsionen geeigneten Kit.

Das invasive Zervixkarzinom geht in der Regel aus einer Präkanzerose hervor. Präkanzerosen umfassen ein breites Spektrum von Läsionen, die histopathologisch als leichte bis schwere Dysplasie (CIN1 bis CIN3) bezeichnet werden. CIN1-Läsionen bilden sich häufig spontan zurück und müssen in der Regel nicht therapiert werden. Andererseits können diese Läsionen über Jahre persistieren oder in eine höhergradige Läsion übergehen, z.B. C1N3 oder in ein mikroinvasives Karzinom. Zur Diagnose von Zervixabstrichen wird seit rund 50 Jahren ein zytologisches Verfahren angewendet, mit welchem dysplastische Zellen in Zervixabstrichen nachgewiesen werden können. Allgemein bekannt ist dieses Verfahren unter dem Begriff "Pap-Test". Der "Pap-Test," hat dazu beigetragen, daß die Inzidenz des Zervixkarzinoms in der Vergangenheit signifikant gesenkt werden konnte.

Vor einigen Jahren wurde weiter gefunden, daß das Vorliegen von dysplastischen Läsionen und Zervixkarzinomen mit dem Nachweis von Zervixkarzinomassoziierten humanen Papillomviren, z.B. HPV 16 oder HPV 18, korreliert. Als Nachweis für prämaligne oder maligne Zervixerkrankungen ist auch der Nachweis von Antikörpern gegen die viralen HPV Onkoproteine E6 und E7 im Serum von Patienten mittels ELISA oder vergleichbaren Methoden möglich. Diskutiert wird auch, daß bestimmte chromosomale Deletionen mit einem erhöhten Risiko zu maligner Transformation der entsprechenden Präkanzerose assoziiert sind. Ebenso korrelieren morphologische Veränderungen von Zellen und Zellkernen mit maligner Progression. Diese Veränderungen können zytometrisch erfaßt werden.

Dennoch ist es weder durch den "Pap-Test" noch durch den Nachweis onkogener HPV-Typen möglich, eine Prognose über die Weiterentwicklung einzelner Läsionen zu geben. Dies gilt auch für den Nachweis HPV-spezifischer Antikörper im Serum von Patienten. Vielmehr ist es mit diesem serologischen Verfahren nur möglich, Patienten zu erfassen, bei denen bereits ein Karzinom vorliegt. Das Antikörper-Nachweis Verfahren kann daher nicht als Ergänzung zur derzeitigen Vorsorge gesehen werden, sondern nur zur Manifestierung des Befundes, daß sich bereits ein Karzinom entwickelt hat. Des weiteren haben die genetischen Analysen und zytometrischen Ansätze den Nachteil, daß sie technisch sehr aufwendig sind und daher in der Routine-Diagnostik keinen Stellenwert erreicht haben.

Der vorliegenden Erfindung liegt somit die Aufgabe zugrunde, ein Mittel bereitzustellen, mit dem das Progressionspotential von Zervixläsionen zuverlässig abgeschätzt werden kann.

Erfindungsgemäß wird dies durch die Gegenstände in den Patentansprüchen erreicht.

Gegenstand der vorliegenden Erfindung ist somit die Verwendung einem Nukleinsäure zur Herstellung eines Diagnosemittels zur Abschätzung des Progressionspotentials von Zervixläsionen. Eine solche Nukleinsäure ist durch ein verfahren erhältlich, bei dem man RNA von frühen und späten Passagen HPV-immortalisierter Zellen isoliert und von dieser RNA jene identifiziert und als DNA oder RNA bereitstellt, die für die frühen bzw. die späten Passagen charakteristisch ist bzw. in deutlich unterschiedlichen Mengen exprimiert ist. Die vorliegende Erfindung betrifft auch die entsprechende Verwendung eines von dieser Nukleinsäure codierten Polypeptids und eines gegen dieses Polypeptides gerichteten AntiKörpers.

Die vorliegende Erfindung beruht auf der Erkenntnis des Anmelders, daß späte Passagen von HPV-immortalisierten Zellen in Nacktmäusen Tumoren hervorrufen, während frühe Passagen solcher Zellen hierzu nicht in der Lage sind. Ferner hat der Anmelder erkannt, daß in späten Passagen HPV-immortalisierter Zellen bestimmte RNAs stärker nachweisbar sind, als dies in frühen Passagen solcher Zellen der Fall ist.

Zur Bereitstellung einer erfindungsgemäßen Nukleinsäure kann RNA von frühen und späten Passagen HPV-immortalisierter Zellen isoliert werden. Frühe Passagen sind z.B. Passagen 20-60, und späte Passagen z.B. ab 130. Als Zellen können z.B. HPV 16-immortalisierte, humane Vorhautkeratinozyten, HPK- lA-Zellen, verwendet werden (vgl. Dürst, M. et al., Oncogene 1/3, (1987), 251-256). Die RNA der frühen und späten Passagen kann; miteinander verglichen werden, wodurch Unterschiede ermittelt werden, die für die frühen bzw. späten Passagen charakteristisch sind. Hierzu ist es günstig, die RNA einer reversen Transkription zu unterziehen. Dabei ist es vorteilhaft, sog. Verankerungsprimer zu verwenden, d.h. oligo-d(T)-Primer, die am 3'-Ende auf eine Folge von 11-15 Thymidin-Basen noch zwei andere Basen aufweisen, und somit gezielt den Übergang vom 3'-Ende einer mRNA in den Poly(A)-Schwanz erkennen und dort binden. Die erhaltene cDNA kann einer Amplifikation in einem PCR-Verfahren unterzogen werden. Hierfür ist es günstig, übliche "Arbitrary"-Primer, zusammen mit vorstehenden Verankerungsprimern zu verwenden. Die amplifizierte cDNA kann dann einer denaturierenden Polyacrylamid-Gelelektrophorese unterworfen werden. In diesem Schritt werden cDNA-Banden identifiziert, die eine unterschiedliche Intensität in den miteinander zu vergleichenden cDNA-Proben, d.h RNA-Isolaten aus den frühen und späten Passagen HPV-immortalisierter Zellen, aufweisen. Diese cDNA-Banden können aus dem Gel isoliert und einer weiteren vorstehenden Amplifikation unterzogen werden. Ferner können sie kloniert und in ihrer Sequenz bestimmt werden. Vorstehende Verfahren sind dem Fachmann bekannt. Ergänzend wird auf die folgende Literatur verwiesen (vgl. Liang et al., Cancer Research 52, (1992), 6966-6968; Liang et al., Science 257, (1992), 967-971; Liang et al., Nucleic Acids Research 21, (1993), 3269-3275).

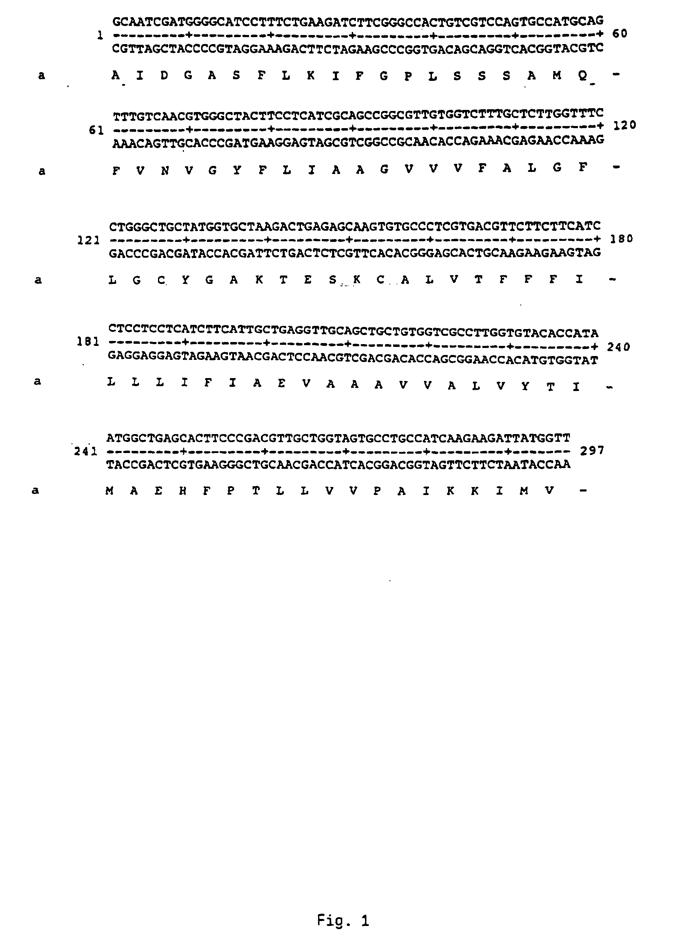

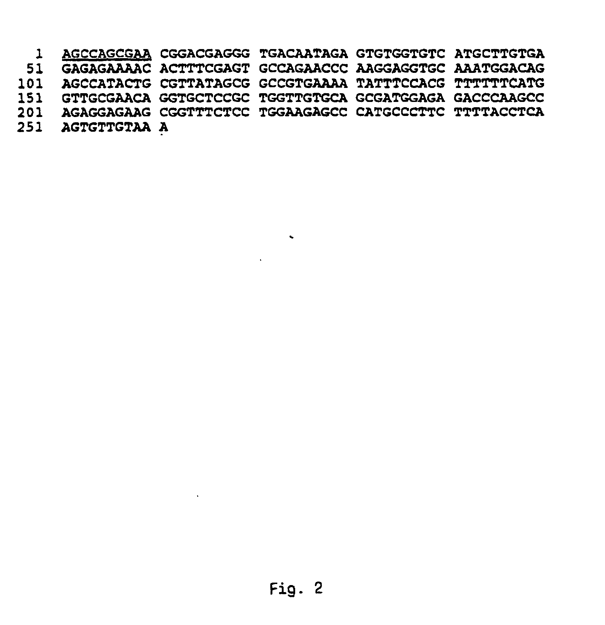

Die vorliegende Erfindung betrifft auch eine zur Abschätzung des Progressions potentials von Zervixlösionen geeignete. Nukleinsäure vorszugweise DNA, welche die Basensequenz von Fig. 1 oder Fig. 2 umfaßt. Die (c)DNA von Fig. 1 wurde als C4,8 bei der DSMZ (Deutsche Sammlung von Mikroorganismen und Zellkulturen) unter DSM 11197 am 04. Oktober 1996 hinterlegt. Ferner wurde die (c)DNA von Fig. 2 als C21,7 bei der DSMZ unter DSM 11198 am 04. Oktober 1996 hinterlegt. Nachstehend wird eine erfindungsgemäße Nukleinsäure beispielhaft als DNA beschrieben.

Eine erfindungsgemäße DNA kann in einem Vektor bzw. Expressionsvektor vorliegen. Beispiele solcher sind dem Fachmann bekannt. Im Falle eines Expressionsvektors für E. coli sind dies z.B. pGEMEX, pUC-Derivate, pGEX-2T, pQE-8 und pet3d. Für die Expression in Hefe sind z.B. pY100 und Ycpad1 zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A.

Der Fachmann kennt geeignete Zellen, um eine, erfindungsgemäße, in einem Expressionsvektor vorliegende DNA zu exprimieren: Beispiele solcher Zellen umfassen die E.coli-Stämme HB101, DH1, x1776, JM101, JM 109, SG 13009 und BL21, den Hefe-Stamm Saccharomyces cerevisiae und die tierischen Zellen L, 3T3, FM3A, CHO, COS, Vero und HeLa sowie die lnsektenzeilen sf9.

Der Fachmann weiß, in welcher Weise eine erfindungsgemäße DNA in einen Expressionsvektor inseriert werden muß. Ihm ist auch bekannt, daß diese DNA in Verbindung mit einer für ein anderes Polypeptid kodierenden DNA inseriert werden kann, so daß die erfindungsgemäße DNA in Form eines Fusionspolypeptids exprimiert werden kann.

Des weiteren kennt der Fachmann Bedingungen, transformierte bzw. transfizierte Zellen zu kultivieren. Auch sind ihm Verfahren bekannt, das durch die erfindungsgemäße DNA exprimierte Polypeptid zu isolieren und zu reinigen. Ein solches Polypeptid, das auch ein Fusionspolypeptid sein kann, ist somit ebenfalls Gegenstand der vorliegenden Erfindung und umfaßt die Aminosäuresequenz von Fig. 1.

Ein weiterer Gegenstand der vorliegenden Erfindung ist ein gegen ein vorstehendes Polypeptid bzw. Fusionspolypeptid gerichteter Antikörper. Ein solcher Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit einem vorstehenden (Fusions)potypeptid zu immunisieren. Weitere "Booster" der Tiere können mit dem gleichen (Fusions)polypeptid erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.

Die vorliegende Erfindung ermöglicht es, das Progressionspotential von Zervixläsionen zuverlässig abzuschätzen. Mit einem erfindungsgemäßen Antikörper kann in Zervixabstrichen festgestellt werden, ob diese Polypeptide enthalten, die für frühe oder späte Passagen HPV-immortalisierter Zellen charakteristisch sind. Ferner kann mit einem erfindungsgemäßen Polypeptid ein gegen das im Körper vorliegende Polypetid gerichteter Autoantikörper nachgewiesen werden. Beide Nachweise können durch übliche. Verfahren, insbesondere einen Western Blot, einen ELISA, eine Immunpräzipitation oder durch Immunfluoreszenz, erfolgen. Desweiteren kann mit einer erfindungsgemäßen Nukleinsäure, insbesondere einer DNA und hiervon abgeleiteten Primern, in Zervixabstrichen festgestellt werden, ob hier RNA vorliegt, die für frühe oder späte Passagen HPV-immortalisierter Zellen charakteristisch ist. Dieser Nachweis kann in üblicher Weise, insbesondere in einem Southern Blot, erfolgen. Mit der vorliegenden Erfindung ist es somit möglich, frühzeitig eine Aussage zu machen, ob sich ein Zervixkarzinom im Entstehen befindet.

Darüber hinaus eignet sich die vorliegende Erfindung, Maßnahmen gegen das Entstehen eines Zervixkarzinoms zu ergreifen. Mit einem erfindungsgemäßen Antikörper kann ein Polypeptid inhibiert werden, das für späte Passagen HPV-immortalisierter Zellen charakteristisch ist. Auch kann eine erfindungsgemäße Nukleinsäure, insbesondere eine DNA, zur Inhibierung eines solchen Polypeptids genutzt werden. Hierzu wird die Nukleinsäure, z.B. als Basis für die Erstellung von Anti-Sinn-Oligonukleotiden zur Expressions-Inhibierung des für das Polypeptid kodierenden Gens verwendet.

Zur Durchführung der vorliegenden Erfindung, insbesondere hinsichtlich des diagnostischen Aspekts, wird weiterhin ein Kit bereitgestellt. Dieser enthält ein oder mehrere erfindungsgemäße Nukleinsäuren, Polypeptide und/oder Antikörper. Insbesondere umfaßt er solche Nukleinsäuren und/oder Polypeptide, die vorstehend als bevorzugt genannt sind. Ferner enthält der Kit übliche Hilfsstoffe, wie Träger, Puffer, Lösungsmittel und Kontrollen. Der Kit ist ebenfalls Gegenstand der vorliegenden Erfindung.

Die vorliegende Erfindung wird durch die nachstehenden Beispiele erläutert.

Kurze Beschreibung der Zeichnungen:
- **Fig. 1**: zeigt die Basensequenz der erfindungsgemäßen (c)DNA C4,8. Ferner ist die Aminosäuresequenz des durch die (c)DNA C4,8 codierten Polypeptids angegeben,
- **Fig. 2**: zeigt die Basensequenz der erfindungsgemäßen (c)DNA C21,7.

### BEISPIEL 1: Herstellung von erfindungsgemäßen cDNAs, C4,8 und C21,7

Aus frühen Passagen d.h. Passage p49, und späten Passagen, d.h. Passage p359 und p389, der HPV-immortalisierten Zellinie HPK-lA (vgl. vorstehend), wurde jeweils Gesamt-RNA durch das bekannte Guanidin-Thiocyanat (GTC)-Verfahren isoliert. Die Gesamt-RNA wurde einer üblichen DNase-Reaktion unterzogen, wobei die RQ1-RNase-freie DNase von PROMEGA verwendet wurde.

Die erhaltene DNase-freie Gesamt-RNA wurde einem reversen Transkriptions-Verfahren unterworfen, wobei als Primer sog. Verankerungsprimer verwendet wurden. Diese Primer weisen am 3'-Ende auf eine Folge von 11-15 Thymidin-Basen noch zwei andere Basen auf, z.B. AA, AC, AG, CA, CC, CG, GA, GC, GG, AT, CT, GT, wodurch die Bindung der Primer direkt am Übergang der mRNA auf den Poly(A)-Schwanz erfolgt.

Die Bedingungen für die reverse Transkription waren wie folgt:

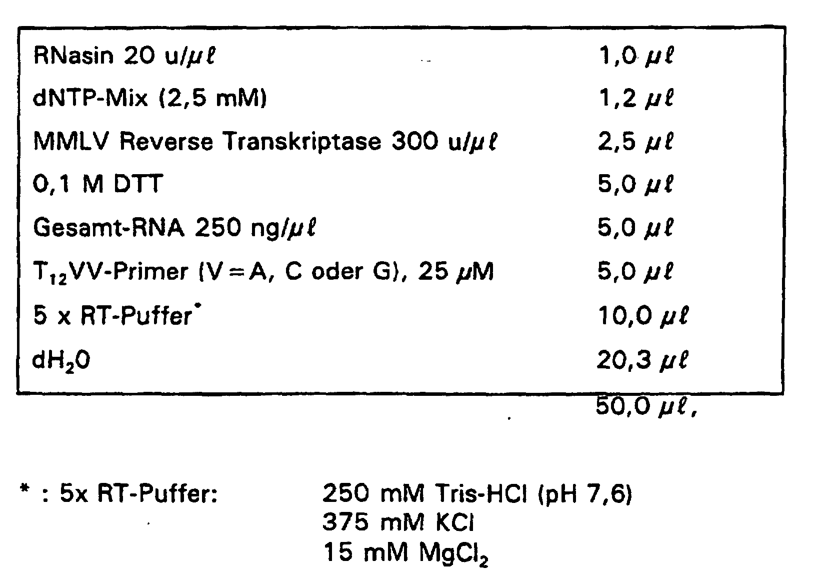

Die Gesamt-RNA wurde vor der Reaktion 5 Minuten bei 70-80 °C denaturiert, dann auf Eis abgeschreckt und im Reaktionsgefäß vorgelegt. Alle restlichen Komponenten wurden bei 0 °C gemischt und zur Gesamt-RNA gegeben, zuletzt wurde alles mit Mineralöl überschichtet und 45-60 Minuten bei 37 °C im Wasserbad inkubiert. Schließlich wurde die Reaktion durch Inaktivierung des Enzyms bei 95 °C (5 Minuten) gestoppt. Nach Beendigung der Reaktion wurden die RT-Ansätze bei -20 °C bis zum Gebrauch eingefroren.

Die erhaltene cDNA wurde einem PCR-Verfahren unterworfen. Hierzu wurden 20 µℓ-Ansätze gemacht, die jeweils 2 µℓ des vorstehenden reverse Transkriptionsansatzes als Template (1 /10 des Reaktionsvolumens, entsprechend dem Äquivalent von 25-50 ng cDNA/PCR-Ansatz) aufwiesen. Die restlichen Komponenten (vg. nachstehend) wurden als "Master-Mix" angesetzt und anschließend zugegeben. Ein PCR-Reaktionsansatz setzte sich wie folgt zusammen:

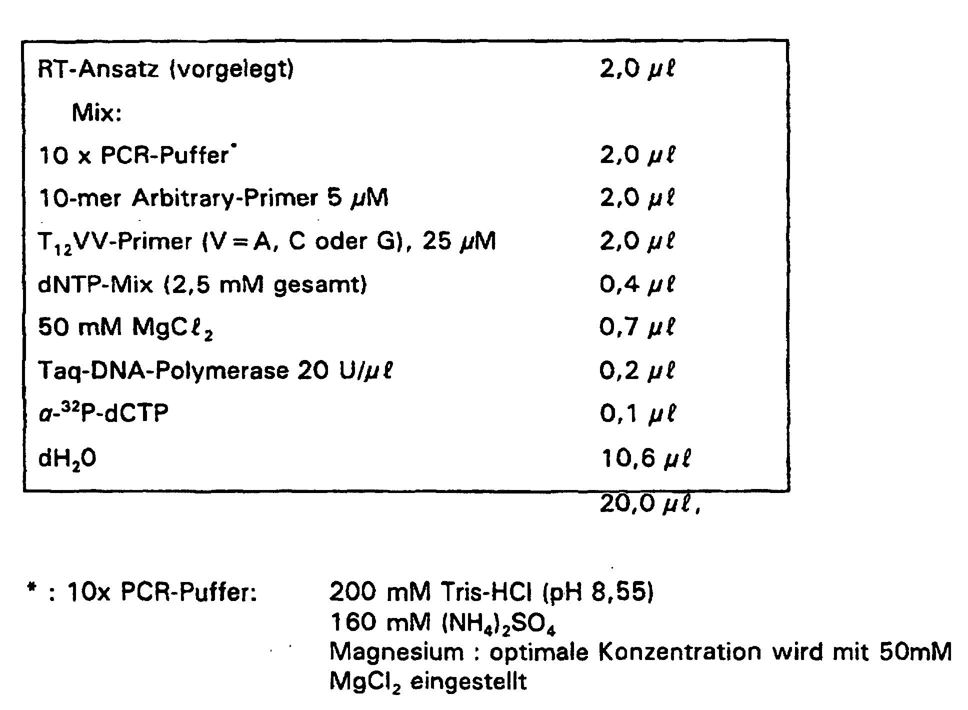

Der 10-mer Arbitrary-Primer ist z.B. "AGC CAG CGA A" (AP-1) oder "GCA ATC GAT G" (AP-6). Die Reaktion wurde im DNA-Thermocycter (Perkin-Elmer Gen-Amp 9600) mit folgenden Programmschritten durchgeführt:

| | | | |
|---|---|---|---|
| Programm 1: | Denaturierung | 95 °C, 3 Minuten | 1 Zyklus |
| Programm 2: | Denaturierung | 95 °C, 15 Sekunden | |
| | Primer-Annealing | 40 °C, 2 Minuten | max. 30 Zyklen |
| | Primer-Extension | 72 °C, 30 Sekunden | |
| Programm 3: | Primer-Extension | 72 °C, 5 Minuten | 1 Zyklus |

Nach Beendigung des PCR-Verfahrens wurden die Ansätze auf ein denaturierendes, 4,5-6 % Polycrylamidgel aufgetragen. Durch Vergleich der cDNA-Banden aus den frühen und späten Passagen der HPK-lA-Zellen konnten diejenigen identifiziert werden, die unterschiedlich waren, d.h. in den späten Passagen viel stärker vertreten waren als in den frühen.

Diese cDNA-Banden wurden für eine weitere Amplifikation verwendet. Hierzu wurden sie aus dem Polyacrylamidgel herausgeschnitten und in ein weiteres PCR-Verfahren eingebracht. Der PCR-Ansatz setzte sich wie folgt zusammen:

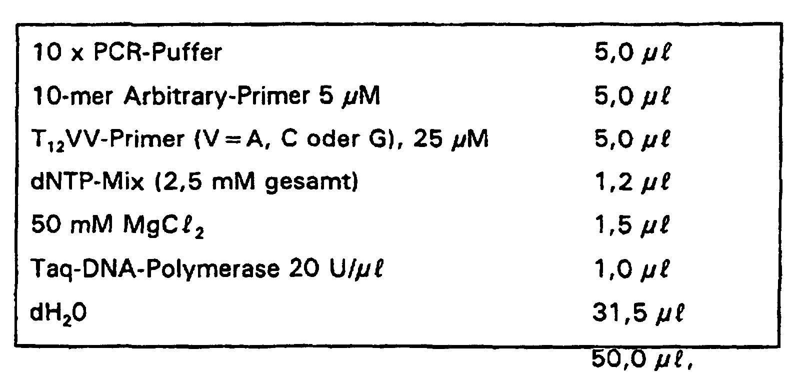

Die PCR-Reaktion wurde unter den gleichen Bedingungen und mit derselben Abfolge von Programmen wie die erste PCR-Reaktion durchgeführt.

Nach Beendigung der PCR-Reaktion wurden die Ansätze auf einem 1 % Agarosegel aufgetrennt, und die gewünschten DNA-Banden aus dem Gel herausgeschnitten. Anschließend wurden die DNA-Banden aus den Agarose-Stückchen eluiert, wobei sog. "GenElute"-Säulchen (SUPELCO) verwendet wurden.

Die erhaltene cDNA wurde in dem Klonierungsvektor pCRll unter Verwendung des "TA-Kloning-Kit" (INVITROGEN) kloniert. Erhaltene Klone wurden mittels des "T7-Sequencing-Kit" (PHARMACIA) in ihrer Sequenz bestimmt. Es wurden die erfindungsgemäßen cDNAs, C4,8 und C21,7 erhalten.

### Beispiel 2: Vergleichsstudien unter Verwendung der erfindungsgemäßen cDNAs C4,8 und C21,7

a) Die gemäß Beispiel 1 isolierte Gesamt-RNA aus frühen und späten Passagen von HPK-lA Zellen wurde einer denaturierenden 4,5-6 % Polyacrylamid- Gelelektrophorese unterworfen. Anschließend wurde ein üblicher Northern-Blot durchgeführt, wobei die RNA auf "Gene Screen-Plus"-Nylonmembranen transferierte wurde. Zur Hybridisierung wurden erfindungsgemäße ³²P-markierte cDNAs, C4,8 und C21,7, verwendet.
   Es zeigte sich, daß die erfindungsgemäßen cDNAs weit stärker mit der RNA aus den späten Passagen der HPK-1A-Zellen reagierten, als dies mit den frühen Passagen der Fall war.
b) Ferner wurden RNA-RNA- insitu Hybridisierungen an Gefrierschnitten von Zervixgewebe, nämlich normalem Epithelgewebe, prämaligner Läsion und Karzinom, vorgenommen, um den Zustand des Gewebes abzuschätzen. Als Hybridisierungssonden wurden RNA-Proben verwendet, die von den erfindungsgemäßen cDNAs C4.8 und C21.7 erhalten wurden. Dazu wurden letztere linearisiert und RNA wurde durch Zugabe der entsprechenden RNA-Polymerase, bevorzugt SP6 oder T7, und ³²P-rUTP synthetisiert. Die RNA-RNA in situ Hybridisierung mit dem vorstehenden Gewebe wurde unter stringenten Bedingungen durchgeführt, z.B. bei 60°C.
   Es zeigte sich, daß nur beim Zervixkarzinom eine starke Hybridisierung erhalten wurde. Bei normalen Epithelgewebe war dagegen die Hybridisierung äußerst gering.

Die vorstehenden Daten unterstreichen, daß sich die vorliegende Erfindung bestens eignet, potentiell maligne Zellen in einem Zervixabstrich zu detektieren.

## Patentansprüche

1. Verwendung einer Nukleinsäure zur Herstellung eines Diagnosemittels zur Abschätzung des Progressionspotentials von Zervixläsionen, wobei die Nukleinsäure durch ein Verfahren erhältlich ist, bei dem man RNA aus frühen und späten Passagen HPV-Immortalisierter Zellen isoliert und von dieser RNA jene identifiziert und als DNA oder RNA bereitstellt, die für die frühen bzw. späten Passagen charakteristisch ist.

2. Verwendung nach Anspruch 1, wobei die Nukleinsäure als DNA bereitgestellt ist.

3. Verwendung nach Anspruch 1 oder 2, wobei die Nukleinsäure die Basensequenz von Fig. 1 umfaßt.

4. Verwendung nach Anspruch 1 oder 2, wobei die Nukleinsäure die Basensequenz von Fig. 2 umfaßt.

5. Verwendung eines Polypeptids zur Herstellung eines Diagnosemittels zur Abschätzung des Progressionspotentials von Zervixläsionen, wobei das Polypeptid von einer in einem der Ansprüche 1 - 4 definierten Nukleinsäure codiert ist.

6. Verwendung eines Antikörpers zur Herstellung eines Diagnosemittels zur Abschätzung des Progressionspotentials von Zervixläsionen, wobei der Antikörper gegen ein Polypeptid gerichtet ist, das von einer in einem der Ansprüche 1 - 4 definierten Nukleinsäure codiert ist.

7. Verfahren zur Herstellung der Nukleinsäure wie in Anspruch 1 definiert, bei dem man RNA aus frühen und späten Passagen HPV-immortalisierter Zellen isoliert und von dieser RNA jene identifiziert und als DNA oder RNA bereitstellt, die für die frühen bzw. späten Passagen charakteristisch ist.

8. Verwendung einer in einem der Ansprüche 1 - 4 definierten Nukleinsäure oder des in Anspruch 6 definierten Antikörpers zur Herstellung eines Arzneimittel zur Behandlung einer Zervixläsion.

9. Nukleinsäure, geeignet zur Abschätzung des Progressionspotentiats von Zervixläsionen, wobei die Nukleinsäure die Basensequenz von (a) Fig: 1 bzw. des Klons DSMZ 11197 oder (b) Fig. 2 bzw. des Klons DSMZ 11198 umfaßt.

10. Nukleinsäure nach Anspruch 9, **dadurch gekennzeichnet, daß** die Nukleinsäure als DNA bereitgestellt ist.

11. Polypeptid, umfassend eine Aminoäsuresequenz, die durch die Nukleinsäure nach Anspruch 9(a) codiert ist.

12. Antikörper, gerichtet gegen das Polypeptid nach Anspruch 11.

13. Kit, umfassend ein oder mehrere Nukleinsäuren nach Anspruch 9, Polypeptide nach Anspruch 11 und/oder Antikörper nach Anspruch 12 sowie übliche Hilfsstoffe.

## Claims

1. Use of a nucleic acid for the production of a diagnostic agent for evaluating the progression potential of cervical lesions, wherein the nucleic acid is obtainable by a process in which RNA is isolated from early and late passages of HPV-immortalized cells and of said RNA the one characteristic of the early and/or late passages is identified and provided as DNA or RNA.

2. Use according to claim 1, wherein the nucleic acid is provided as DNA.

3. Use according to claim 1 or 2, wherein the nucleic acid comprises the base sequence of fig. 1.

4. Use according to claim 1 or 2, wherein the nucleic-acid comprises the base sequence of fig. 2.

5. Use of a polypeptide for the production of a diagnostic agent for evaluating the progression potential of cervical lesions, the polypeptide being encoded by the nucleic acid defined in any of claims 1 to 4.

6. Use of an antibody for the production of a diagnostic agent for evaluating the progression potential of cervical lesions, wherein the antibody is directed against a polypeptide encoded by a nucleic acid defined in any of claims 1 to 4.

7. A process for the preparation of the nucleic acid as defined in claim 1, in which RNA is isolated from early and late passages of HPV-immortalized cells and of said RNA the one characteristic of the early and/or late passages is identified and provided as DNA or RNA.

8. Use of a nucleic acid defined in any of claims 1 to 4 or of the antibody defined in claim 6 for the production of a medicament for treating a cervical lesion.

9. Nucleic acid suited for evaluating the progression potential of cervical lesions, wherein the nucleic acid comprises the base sequence of (a) figure 1 and/or clone DSMZ 11197 or (b) figure 2 and/or clone DSMZ 11198.

10. Nucleic acid according to claim 9, **characterized in that** the nucleic acid is provided as a DNA.

11. Polypeptide comprising an amino acid sequence encoded by the nucleic acid according to claim 9(a).

12. Antibody directed against the polypeptide according to claim 11.

13. Kit comprising one or more nucleic acids according to claim 9, polypeptide according to claim 11 and/or antibody according to claim 12 as well as conventional auxiliary agents.

## Revendications

1. Utilisation d'un acide nucléique pour la préparation d'un produit de diagnostic destiné à évaluer le potentiel de progression de lésions cervicales, l'acide nucléique étant obtenu par un procédé dans lequel on isole de l'ARN de passages précoces et tardifs de cellules immortalisées par un virus HPV et on identifie de cet ARN et rend disponible comme ADN ou ARN celui qui est caractéristique des passages précoces ou respectivement des passages tardifs.

2. Utilisation suivant la revendication 1, dans laquelle l'acide nucléique est rendu disponible comme ADN.

3. Utilisation suivant la revendication 1 ou 2, dans laquelle l'acide nucléique comprend la séquence de bases de la fig. 1.

4. Utilisation suivant la revendication 1 ou 2, dans laquelle l'acide nucléique comprend la séquence de bases de la fig. 2.

5. Utilisation d'un polypeptide pour la préparation d'un produit de diagnostic destiné à évaluer le potentiel de progression de lésions cervicales, le polypeptide étant codé par un acide nucléique défini dans l'une des revendications 1 à 4.

6. Utilisation d'un anticorps pour la préparation d'un produit de diagnostic destiné à évaluer le potentiel de progression de lésions cervicales, l'anticorps étant dirigé contre un polypeptide qui est codé par un acide nucléique défini dans l'une des revendications 1 à 4.

7. Procédé de production de l'acide nucléique suivant la revendication 1, dans lequel on isole de l'ARN de passages précoces et tardifs de cellules immortalisées par un virus HPV et on identifie de cet ARN et rend disponible comme ADN ou comme ARN celui qui est caractéristique des passages précoces ou respectivement des passages tardifs.

8. Utilisation d'un acide nucléique défini dans l'une des revendications 1 à 4 ou de l'anticorps défini dans la revendication 6 pour la préparation d'un médicament destiné au traitement d'une lésion cervicale.

9. Acide nucléique, convenant pour l'évaluation du potentiel de progression de lésions cervicales, l'acide nucléique comprenant la séquence de bases (a) de la fig. 1, plus précisément du clone DSMZ 11197, ou (b) de la fig. 2, plus précisément du clone DSMZ 11198.

10. Acide nucléique suivant la revendication 9, **caractérisé en ce qu'**il est rendu disponible comme ADN.

11. Polypeptide, comprenant une séquence d'amino-acides qui est codée par l'acide nucléique suivant (a) de la revendication 9.

12. Anticorps, dirigé contre le polypeptide suivant la revendication 11.

13. Nécessaire comprenant un ou plusieurs acides nucléiques suivant la revendication 9, polypeptides suivant la revendication 11 et/ou anticorps suivant la revendication 12, ainsi que des substances auxiliaires usuelles.
